# NEUE EUROPÄISCHE PATENTSCHRIFT

(11) **EP 0 579 952 B2**
(45) Veröffentlichungstag und Bekanntmachung des Hinweises auf die Entscheidung über den Einspruch: **30.08.2000**
(45) Hinweis auf die Patenterteilung: 16.08.1995
(21) Anmeldenummer: 93109586.3
(22) Anmeldetag: 16.06.1993
(51) Int. Cl.: G01N 1/26, B08B 9/46, G01N 1/00, B08B 101/08

(54) **Verfahren und Vorrichtung zur Prüfung von Flaschen auf Verunreinigungen**
Method and device for testing of bottles for contamination
Procédé et dispositif pour contrôler l'état de propieté de bouteilles

(30) Priorität: 09.07.1992 CH 216692; 01.09.1992 CH 271592; 08.02.1993 CH 38293
(43) Veröffentlichungstag der Anmeldung: 26.01.1994
(73) Patentinhaber: ELPATRONIC AG, 8962 Bergdietikon (CH)
(72) Erfinder: Gysi, Peter, CH-5454 Bellikon (CH); Zumbach, Melchior, Dr., CH-8600 Dübendorf (CH); Huesser, Theo, CH-8964 Rudolfstetten (CH)

(56) Entgegenhaltungen:
- DE-A- 1 598 415
- DE-A- 3 623 327
- DE-U- 8 806 138
- FR-A- 1 344 848
- US-A- 2 333 934
- US-A- 2 418 691
- US-A- 3 321 954
- US-A- 4 791 820

## Beschreibung

Die Erfindung betrifft ein Verfahren zum Prüfen von entlang einer Förderstrecke geförderten Flaschen, gemäss Obergriff des Anspruchs 1 sowie eine Vorrichtung zu dessen Durchführung gemäss Oberbegriff des Anspruchs 6.

Bei Mehrwegflaschen, insbesondere bei Kunststoff-Flaschen, wie z.B. PET-Flaschen, welche nicht bei hohen Temperaturen gewaschen werden können, stellt sich das Problem, das Verunreinigungen sicher erkannt werden müssen, damit verunreinigte Flaschen ausgeschieden, bzw. der erneuten Befüllung entzogen werden können. Insbesondere müssen dabei zurückgegebene Flaschen erkannt werden können, die ein Benützer für potentiell gefährliche Stoffe (Gifte, Lösungsmittel) verwendet hat. Es ist bereits bekannt, zu diesem Zweck jeweils eine Gasprobe aus der Flasche zu entnehmen und diese durch Flammen-Ionisations-Detektion (US-A-3321954) oder durch Photo-Ionisations-Detektion (PID) zu untersuchen. Damit kann das Vorhandensein auch geringer Spuren unerwünschter Stoffe in der Flasche bzw. im Kunststoff-Material erkannt werden. Da solche Prüfvorrichtungen einen hohen Flaschendurchsatz pro Minute (erwünscht z.B. 250 bis 300 Flaschen pro Minute) aufweisen müssen, damit eine derartige Prüfung in einem industriellen Abfüllbetrieb vorgenommen werden kann, kamen bisher jeweils eine grosse Zahl einzelner PID-Geräte zum Einsatz, d.h. jeder von mehreren zu prüfenden Flaschen wurde ein eigenes PID-Gerät zugewiesen. Dies bedingt indes einen hohen Kosten-, Wartungs- und Kalibrieraufwand.

Der Erfindung liegt deshalb die Aufgabe zugrunde, ein Verfahren zu schaffen, welches den Aufwand an Kosten, Wartung und Kalibrierung deutlich verringert. Dies wird bei einem Verfahren der eingangs genannten Art durch die kennzeichnenden Merkmale des Anspruchs 1 erreicht.

Dadurch, dass Gasproben aus mehreren gleichzeitig in der Prüfung befindlichen Flaschen nacheinander nur einer Prüfeinrichtung zugeführt werden, ergibt sich die gewünschte Reduktion des Aufwandes.

Dies erlaubt es bei einer bevorzugten Ausführungsart als Prüfeinrichtung ein Massenspektrometer zu verwenden. Dieses erlaubt eine besonders gute Erkennung allfälliger Verunreinigungen.

Bei dieser Lösung ergibt sich ein längerer Transportweg für die einzelne Gasprobe von der jeweiligen Flasche zu der mehreren Flaschen gemeinsamen Prüfeinrichung als bei der bekannten Lösung mit einer Vielzahl von jeweils nahe bei den einzelnen Flaschen angeordneten PID-Geräten. Es hat sich jedoch gezeigt, dass entgegen den anfänglichen Erwartungen trotzdem auch bei dem gewünschten hohen Flaschendurchsatz genügend Messzeit für die Prüfung, insbesondere für die massenspektrometische Messung verbleibt.

Erfindungsgemäss werden aber Massnahmen getroffen, um den Einfluss des Transportweges der Gasprobe von der jeweiligen Flasche bis zur Prüfeinrichtung zu verringern bzw. aufzuheben. Insbesondere wird aus jeder zu prüfenden Flasche kontinuierlich Gas zu einer Verteileinrichtung gefördert, welche relativ nahe bei der Prüfeinrichtung, z.B. beim Massenspektrometer angeordnet sein kann. Von den dort zugeführten Gasströmen kann dann jeweils einer auf kurzem Weg zum Massenspektrometer durchgeschaltet werden.

Bevorzugterweise werden ferner zumindest die Leitungen von der Flasche bis zur Verteileinrichtung beheizt, um Kondensation zu vermeiden, und es wird Luft in die zu prüfenden Flaschen eingeblasen, um die Gasprobe möglichst mit eventuellen Verunreinigungen anzureichern.

Die Vorrichtung zur Durchführung des Verfahrens ist gemäss Anspruch 6 definiert.

Im folgenden werden Ausführungsbeispiele der Erfindung anhand der Zeichnungen näher erläutert. Dabei zeigt:
Figur 1 eine schematische Darstellung einer Vorrichtung zur Durchführung des Verfahrens;
Figur 2 eine Darstellung ähnlich Figur 1 zur Erläuterung einer weiteren Ausführungsform des Verfahrens;
Figur 3 eine Draufsicht auf eine Vorrichtung zur Durchführung des Verfahrens mit mehreren Fördermitteln für die Flaschen;
Figur 4 eine teilweise geschnittene Seitenansicht eines Teils der Vorrichtung nach Figur 3;
Figur 5 ein Blockschema der Prüfvorgänge in der Vorrichtung nach Figur 3.
Figur 6 eine teilweise geschnittene Darstellung einer gegenüber Figur 4 geänderten Verteileinrichtung;
Figur 7 eine Draufsicht auf eine der Dichtflächen von Figur 6; und
Figur 8 eine Draufsicht auf eine anders gestaltete Dichtfläche.

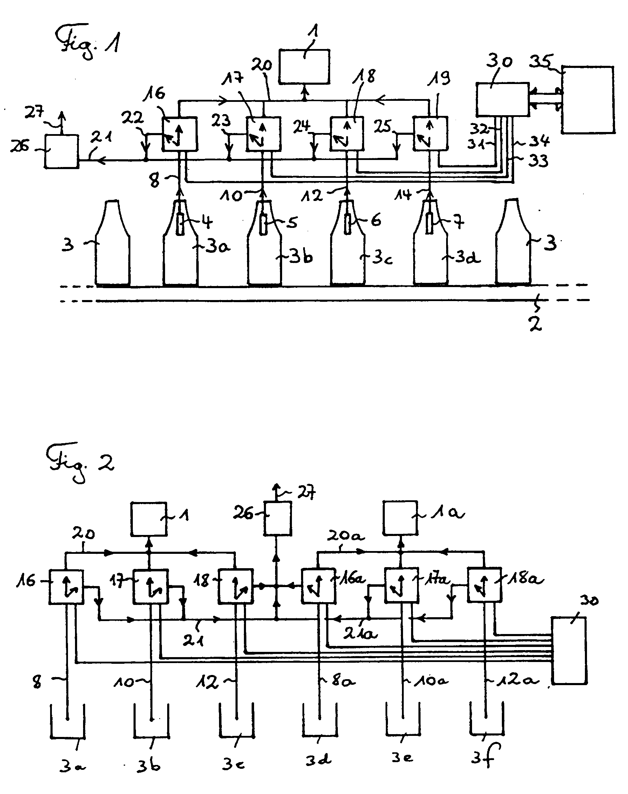

Die in Figur 1 schematisch dargestellte Vorrichtung weist eine Förderstrecke 2 für Flaschen 3 auf. In einem Prüfabschnitt der Föderstrecke 2 werden Gasproben aus den Flaschen 3a, 3b, 3c und 3d entnommen. Dazu werden Probeentnahme-Sonden 4, 5, 6 und 7 durch den Flaschenhals jeweils in die Flaschen eingefahren, ohne die Flasche zu berühren, um jegliche Kontamination auszuschliessen. Durch die Sonden wird Gas aus jeder Flasche abgesaugt und jeweils über eine Leitung 8, 10, 12 und 14 an eine Verteileinrichtung mit den Umschaltventilen 16, 17, 18 und 19 geführt. In der einen Stellung der Umschaltventile wird von diesen das aus der jeweiligen Flasche angesaugte Gas über Ausgangsleitungen 22, 23, 24 und 25 an eine Sammelleitung 21 abgegeben, welche mit einer Saugpumpe 26 in Verbindung steht, welche Saugpumpe 26 das Gas aus der jeweiligen Flasche fördert und über eine Leitung 27 an die Umgebung abgibt. Während der Anwesenheit der Flaschen 3a bis 3d in der Prüfstrecke wird also kontinuierlich durch die Pumpe 26 Gas aus den Flaschen abgesaugt und zu den Umschaltventilen gefördert. Durch eine Steuereinrichtung 30, welche mit einer übergeordneten Steuerung 35 verbunden sein kann, wird nun jeweils über die elektrischen Steuerleitungen 31 - 34 eines der Ventile 16 bis 19 für eine vorbestimmte Zeit umgeschaltet, so dass das Gas aus der jeweiligen Flasche, welches durch das Ventil hindurch strömt, an eine zweite Sammelleitung 20 angelegt wird, welche mit dem Eingang der Prüfeinrichtung, z. B. eines PID-Gerätes, vorzugsweise aber eines Massenspektrometers 1 verbunden ist. Das Ansaugen durch die Leitung 20 zum Massenspektrometer 1 erfolgt durch dessen eigene Förderpumpe. Nachdem durch das Massenspektrometer das Gas der einen zu prüfenden Flasche, z.B. der Flasche 3a, angesaugt worden ist, wird das Ventil 16 wieder umgeschaltet, so dass der Gasstrom aus der Flasche 3a wieder an die Ausgangsleitung 22 durchgeschaltet wird. Sobald das Massenspektrometer 1 für die nächste Analyse, d.h. für die Analyse des Gasstromes aus der Flasche 3b, bereit ist, wird das Ventil 17 umgeschaltet, so dass der Gasstrom aus der Flasche 3b kurzzeitig an die Sammelleitung 20 und somit an das Massenspektrometer 1 abgegeben wird. Auf die gleiche Weise werden nachfolgend nacheinander die Ventile 18 und 19 kurzzeitig umgeschaltet, so dass nacheinander jeweils Gasproben aus den Flaschen 3c und 3d analysiert werden können. Danach werden dann die Sonden 4 bis 7 aus den Flaschen 3a bis 3d ausgefahren, und es werden vier neue Flaschen auf der Förderstrecke 2 bereitgestellt, und die Sonden werden in die neuen Flaschen eingefahren und aus diesen werden ebenfalls Gasproben entnommen. Die vorgängig geprüften Flaschen 3a bis 3d werden auf der Förderstrecke weiter gefördert und diejenigen dieser Flaschen, bei welchen durch das Massenspektrometer 1 eine unzulässige Verunreinigung entdeckt worden ist, werden durch eine Auswurfeinrichtung von der Förderstrecke entfernt. Die geprüften und nicht verunreinigten Flaschen werden dann nach Durchlaufen mindestens einer Waschanlage an eine Füllanlage weitergegeben, wo sie erneut mit einem Getränk gefüllt werden. Mit einer Vorrichtung bzw. mit dem Verfahren wie es anhand der Figur 1 erläutert worden ist, können also mehrere Flaschen mit nur einem Prüfgerät bzw. Massenspektrometer geprüft werden. Damit dies auch bei einer grossen Förderleistung der Förderstrecke 2 möglich ist, wird das Verfahren so durchgeführt, wie anhand der Figur 1 beschrieben, dass nämlich kontinuierlich aus jeder Flasche ein Gasstrom gefördert wird und an eine Verteileinrichtung 16 bis 19 abgegeben wird. Auf diese Weise fällt die Transportzeit für die Gasproben von den Sonden 4 bis 7 durch die Leitungen 8 bis 14 für die Messung nicht ins Gewicht. Für das Massenspektrometer 1 steht die jeweilige Gasprobe am Ausgang der Verteileinrichtung, bzw. der Umschaltventile 16 bis 19 zur Verfügung, d.h. nur noch der Förderweg durch die Leitung 20 zum Massenspektrometer geht in die Messzeit ein. Auf diese Weise kann auch eine grosse Anzahl von Flaschen, z.B. 16 Flaschen bei schneller Förderung auf der Förderstrecke 2 mit nur einem Massenspektrometer 1 analysiert werden.

Weitere Massnahmen zur Verbesserung einer schnellen Analyse können getroffen werden. So ist es z.B. bevorzugt, dass in die Flaschen während der Probeentnahme Luft eingeblasen wird, um die Konzentration der in der Gasprobe enthaltenen Verunreinigungen zu erhöhen. Vorzugsweise kann die Einblasung von Luft dadurch erfolgen, dass die Leitungen 8, 10, 12 und 14 als doppelte Leitungen ausgeführt sind, so dass durch einen Kanal dieser Leitungen wie beschrieben, das Probengas abgesaugt wird und durch den anderen Kanal (mittels einer nicht dargestellten weiteren Pumpe) durch zusätzliche Auslässe in den Sonden 4 bis 7 Luft in die Flaschen eingeblasen wird. Weiterhin hat es sich gezeigt, dass es vorteilhaft ist, mindestens die Probengas-Kanäle der Leitungen 8, 10, 12 und 14 zu beheizen, um eine Kondensation des Probengases an der jeweiligen Leitung zu verhindern. Auch die Zuleitung 20 zum Massenspektrometer 1 kann beheizt sein.

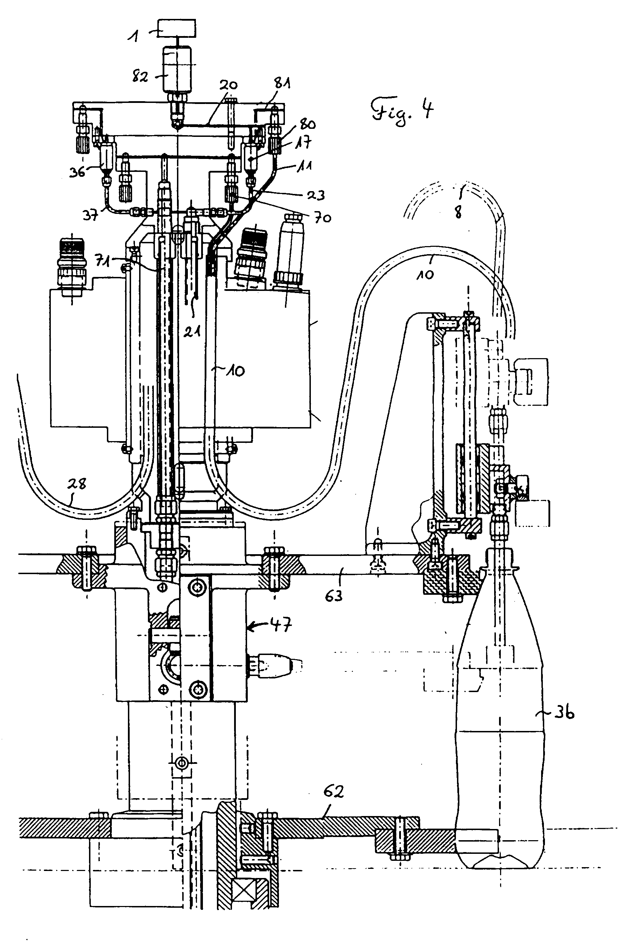

Die Steuereinrichtung 30 steuert einerseits das Umschalten der Ventile 16 bis 19. Weiter kann die Steuereinrichtung 30 auch das Ein- und Ausfahren der Sonden 4 bis 7 aus den jeweiligen Flaschen steuern. Dies kann allerdings auch durch eine übergeordnete Steuerung 35 erfolgen, welche die gesamte Förderanlage für die Flaschen steuert, welche anhand der Figur 3 nachfolgend noch genauer erläutert werden wird. Die Steuerungen 30 und 35 können aber auch als eine kombinierte Steuerung ausgegeführt sein. Das Ein- und Ausfahren der Sonden kann aber auch rein mechanisch mittels steuerkurvenbetätigter Hubeinheiten bewirkt werden.

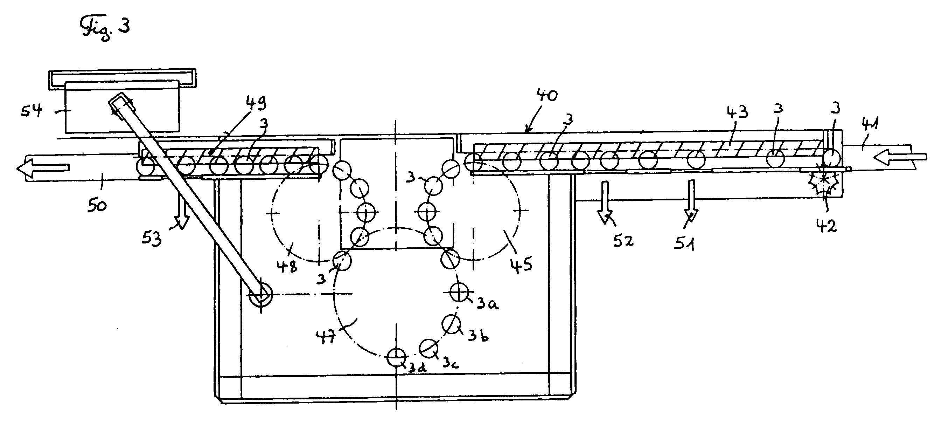

Figur 2 zeigt eine weitere Ausführungsform einer Vorrichtung zur Durchführung des Verfahrens in ebenfalls stark schematisierter Form. Dabei sind zwei Prüfeinrichungen bzw. bevorzugterweise Massenspektrometer 1 bzw. 1a vorgesehen. Das eine Massenspektrometer 1 dient dabei zur Prüfung der Flaschen 3a, 3b und 3c; das andere Massenspektrometer 1a dient zur Prüfung der Flaschen 3d, 3e und 3f. Auch hierbei ist also jeweils ein Massenspektrometer für die Prüfung von mehreren Flaschen gemeinsam zuständig. Die Prüfung der einzelnen Flaschen erfolgt auf analoge Weise wie bei der Figur 1 beschrieben. Zunächst werden also z.B. die Flaschen 3a und 3d jeweils durch das Massenspektrometer 1 bzw. 1a geprüft, indem die Umschaltventile 16 bzw. 16a kurzzeitig den Gasstrom auf die Leitung 20 bzw. 20a umschalten, damit die Gasprobe aus der Flasche 3a durch das Massenspektrometer 1 analysiert werden kann, bzw. die Gasprobe aus der Flasche 3d durch das Massenspektrometer 1a. Nachfolgend erfolgt wieder die Umschaltung der Ventile auf die Sammelleitung 21 bzw. 21a und damit auf die Saugpumpe 26. Nachfolgend findet die Prüfung von Gasproben aus den Flaschen 3b bzw. 3e auf analoge Weise statt.

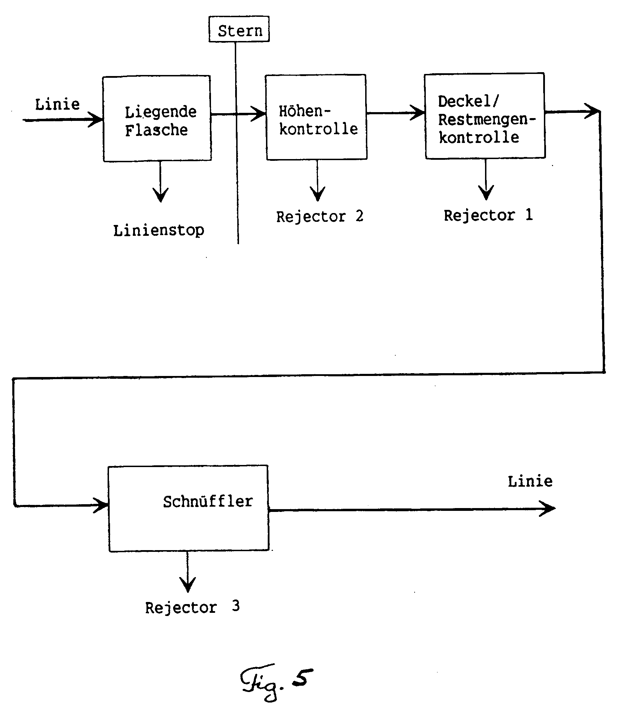

Figur 3 zeigt schematisch eine Draufsicht auf eine nach dem Verfahren arbeitende Vorrichtung 40 zum Prüfen von Flaschen. Zurückkehrende Mehrwegflaschen werden ungeordnet auf einer Förderlinie 41 an die Vorrichtung abgegeben. Die Flaschen werden stehend an die Vorrichtung abgegeben und sind in der Regel offen, d.h. nicht mehr mit einem Deckel versehen und noch nicht gewaschen. Mit einem Conveyor und entsprechendem Staudruck werden die Flaschen 3 einem Kontrollstern 42 zugeführt, der auch den Linienstop bildet, und an eine Förderschnecke 43 abgegeben, welche die Flaschen in regelmässig beabstandeter Reihenfolge stehend weiter fördert. Entlang dieser Förderstrecke 43 können mehrere Prüfanordnungen vorgesehen sein, welche nachfolgend anhand der Figur 5 noch im Detail erläutert werden. Insbesondere können die Flaschen auf korrekte Höhe und auf das Vorhandensein eines Deckels oder eines sonstigen Verschlusses und einer Restmenge von Flüssigkeit geprüft werden. Nicht geeignete Flaschen können durch einen der Auswerfer 51 oder 52 aus der Förderschnecke ausgeworfen werden. Die derart vorgeprüften Flaschen 3 gelangen von der Schnecke 43 auf ein Einlauf-Karussell 45. Von diesem Einlauf-Karussell 45 werden die Flaschen an ein Hauptkarussell 47 abgegeben. Während der Aufenthaltsdauer der Flaschen im Hauptkarrussel 47 erfolgt die Prüfung z.B. als massenspektrometrische Prüfung. In der Figur 3 sind nur schematisch vier Flaschen 3a bis 3d dargestellt. In der Realität wird das Hauptkarussell aber eine grössere Anzahl zu prüfender Flaschen aufnehmen können, z.B. 16 Flaschen, welche von einem oberhalb des Hauptkarrussels angeordneten Massenspektrometer geprüft werden. Nach der Prüfung gelangen die geprüften Flaschen über ein Auslauf-Karussell 48 auf eine Auslaufschnecke 49. Bei dieser Auslaufschnecke ist ein weiterer Auswerfer 53 angeordnet, welcher diejenigen Flaschen auswirft, welche vom Massenspektrometer als verunreinigt erkannt worden sind. Das Auswerfen kann dabei auf verschiedene, bekannte Weisen erfolgen, z.B. durch einen Druckluftstrahl oder durch einen elektromechanisch betätigten Auswurf-Stössel. Bevorzugt ist aber ein "sanftes" Ausleitsystem, welches die auszuscheidenden Flaschen stehend einer anderen Förderstrecke zuführt. Dadurch wird das Umkippen von eventuell schädliche Flüssigkeitsmengen enthaltenden Flaschen vermieden. Diese Flaschen werden stehend an eine Entsorgungsstelle gefördert. Nach der Förderschnecke 49 werden die nicht kontaminierten Flaschen an eine Förderlinie 50 abgegeben, mittels welcher sie an eine Wasch-Station und nachfolgend an eine Befüll-Station abgegeben werden. An einem Ausleger ist ein Bedienschrank 54 angeordnet. Die Steuerung der gesamten Vorrichtung kann separat untergebracht sein.

Figur 4 zeigt eine teilweise geschnittene Teilansicht des Hauptkarussells 47 von Figur 3. Gezeigt ist eine Flasche 3b, welche von Halterungen 62 und 63 des Karussells gehalten wird. Die weiteren im Karussell befindlichen Flaschen und deren Halterungen sind nicht dargestellt. In die eine gezeigte Flasche taucht die Sonde 5 zur Entnahme der Gasprobe ein. Die Sonde 5 ist an einem beweglichen Schlitten 61 gehalten, welcher entlang einer Schlittenführung 60 aus der dargestellten unteren Position in die nur strichliert dargestellte obere Position verfahrbar ist. Beim Eingangsbereich der Flaschen ins Karussell befindet sich jeweils der der Halterung 63 zugeordnete Schlitten 61 in der oberen Position. Nachdem eine Flasche in die Halterung gelangt ist, wird der Schlitten 61 entlang der Schlittenführung 60 nach unten verfahren, wodurch die entsprechende Sonde berührungslos durch den Flaschenhals in die Flasche eintritt. An die Sonde 5 ist ein flexibler Schlauch 10 angeschlossen. Die Anschlussstelle ist zwecks besserer Darstellung in der Zeichnung nicht gezeigt. Bei der unteren Stellung des Schlittens 61 ergibt sich ein Verlauf des flexiblen Schlauches, wie bei demjenigen mit dem Bezugszeichen 10 bezeichneten Schlauch dargestellt. Bei der oberen Stellung des Schlittens 61 ergibt sich ein Verlauf, wie er anhand des kurzen Schlauchabschnittes 8 des hinter dem Schlauch 10 liegenden Schlauchs dargestellt ist. Der Schlauch 10 ist zum Zentrum des Karussells geführt. Er enthält eine Leitung 11, durch welche die Gasprobe aus der Flasche entnommen wird. Die Leitung 11 ist mittels eines Anschlussstücks 80 an das obere Ende des Karussells gekoppelt. Von dem Anschlussstück führt ein Kanal 81 im oberen Ende des Karussells in das Umschaltventil 17. Der eine Ausgang des Umschaltventils ist mit einer Leitung 23 an eine zentrale Leitung 21 des Karussells gekoppelt, welche zu der nicht dargestellten Saugpumpe 26 (Figur 1) führt. Der andere Ausgang des Umschaltventils 17 führt über den Kanal 20 im oberen Karussell-Teil an ein Anschlussstück 82. Die bisher erwähnten Teile des Karussells drehen mitsamt der Flasche mit der Drehgeschwindigkeit des Karussells. Oberhalb des Anschlussstücks 82 ist das feststehende Massenspektrometer 1 angeordnet, welches in der Figur 4 nur schematisch durch einen entsprechenden Block dargestellt ist. Ueber das Anschlussstück 82 ist das feststehende Massenspektrometer mit dem drehenden Karussell, d.h. mit dessen Kanälen 20 verbunden. Die elektrischen Steuerleitungen zu den Umschaltventilen sind in der Figur 4 nicht dargestellt. Diese gelangen von der Steuerung über Schleifkontakte zu den mit dem Karussell drehenden Ventilen. Bei dem in Figur 4 gezeigten Karussell sind z.B. 16 Aufnahmepositionen (Halterungen) für zu prüfende Flaschen vorhanden und ebenso viele Schlitten, Sonden, flexible Schlauchleitungen, Anschlussstücke, elektrische Umschaltventile und Kanäle 20 zum einzigen Anschlussstück 82. Der besseren Darstellung halber sind in Figur 2 indes nur zwei Umschaltventile, das Ventil 17 und das Ventil 36 gezeigt. Auch die restlichen Elemente sind nur teilweise dargestellt, so z.B. die Schläuche 8, 10 und 28. Ueber eine zentrale Leitung 71 und 16 Kanäle 73, bzw. Leitungen 72 und die entsprechenden flexiblen Schläuche, kann in alle zu prüfenden Flaschen saubere Luft eingeblasen werden, um die Konzentration von Verunreinigungen in der entnommenen Gasprobe zu erhöhen. Die flexiblen Schläuche 10 sind ferner beheizt, um eine Kondensation des entnommenen Gases in der Schlauchleitung zu verhindern.

Figur 5 zeigt schematisch die in der Vorrichtung gemäss Figur 3 vorgenommenen Prüfschritte. Die auf der Föderlinie ankommenden Flaschen gelangen zum Kontrollstern 42, wobei liegende Flaschen durch den Stern 42 einen Linienstop herbeiführen. In der Einlaufschnecke findet nachfolgend mittels zweier Lichtschranken eine Kontrolle der Höhe jeder einzelnen Flasche statt. Zu grosse oder zu kleine Flaschen werden ausgeschieden. Als nächstes erfolgt mittels eines Ultraschallsensors eine Kontrolle, ob bei jeder Flasche der Flaschendeckel entfernt ist. Flaschen mit Flaschendeckel oder sonstigem Verschluss werden ausgeschieden. Mittels eines Gewichtssensors erfolgt eine Kontrolle, ob sich noch eine grössere Menge Restflüssigkeit in der Flasche befindet. Ist dies der Fall, so wird die Flasche ausgeschieden.

Nach dem Einlaufmodul gelangen die Flaschen über ein Einlaufkarussel auf das Hauptkarussel. Auf dem Hauptkarussell wird den Flaschen das Probegas entnommen und dem Massenspektrometer oder PID-Prüfgerät für die Messung zugeführt. Die Verweildauer einer Flasche auf dem Karussell ist viel grösser als die Messzeit, welche pro Flasche zur Verfügung steht (zum Vergleich: die Messzeit pro Flasche beträgt ca. 240ms während die Verweildauer auf dem Karussell (bei 300 Flaschen pro Minute) circa 2sec beträgt).

Um die grössere Verweildauer der Flaschen auf dem Karussell auszunützen, geschieht die Probegasentnahme in mehreren Stufen:
0. Stufe
   Sobald die Flasche auf dem Hauptkarussell ist, taucht eine Sonde für die Probegasentnahme in die Flasche ein.
1. Stufe von der Probegasentnahme bis zum Ventilblock
   Alle 16 Probegasschläuche auf dem Hauptkarussell saugen permanent Luft bis zum Ventilblock an. Sobald nun ein Probeschlauch in eine Flasche eintaucht, wird damit das Probegas in der Flasche zum Ventilblock gepumpt. Dort wird es von einem Ventil entweder direkt über die Luftpumpe wieder ins Freie gepumpt, oder aber durch Schalten des Ventils zum Massenspektrometer geleitet.
   Jeweils eines der 16 Ventile auf dem Ven tilblock ist zum Massenspektrometer durchgeschaltet, die anderen sind geschlossen und deren Probegas wird ins Freie geleitet. Zusätzlich zur Entnahme des Probegases wird noch saubere Luft in die Flaschen geblasen. Damit will man eine höhere Konzentration des Probegases erreichen. Schliesslich sind alle Probegasschläuche geheizt, damit ein Kondensieren des Probegases im Schlauch vermieden wird.
2. Stufe vom Ventilblock bis zum Massenspektrometer
   Wie bereits erwähnt ist jeweils eines der 16 Ventile durchgeschaltet. Damit kann das Probegas von dem entsprechenden Probeschlauch und der dazugehörigen Flasche zum Massenspektrometer gelangen und dort analysiert werden. Durch geeignetes, zyklisches Schalten der Ventile kann der Reihe nach jede einzelne Flasche auf dem Karussell analysiert werden.
   Diese 2. Stufe ist sequentiell, d.h. es kann jeweils nur eine Flasche "behandelt" werden, dies im Gegensatz zur 1. Stufe, wo jeweils 15 Stationen parallel, d.h. gleichzeitig "behandelt" werden.

Die Probegasanalyse im Massenspektrum liefert schliesslich das Ergebnis ob eine Flasche kontaminiert ist oder nicht.

Anstelle der beschriebenen elektrisch steuerbaren Verteileinrichtung kann auch eine rein mechanisch, durch die Drehbewegung des Karussels gesteuerte Verteileinrichtung für die Probegasströme vorgesehen sein.

Anstelle des beschriebenen Karussells kann die Prüfung auch entlang eines oder mehrerer paralleler oder serieller linearer Förderwegabschnitte vorgenommen werden, wie dies im Prinzip in den Figuren 1 und 2 dargestellt ist.

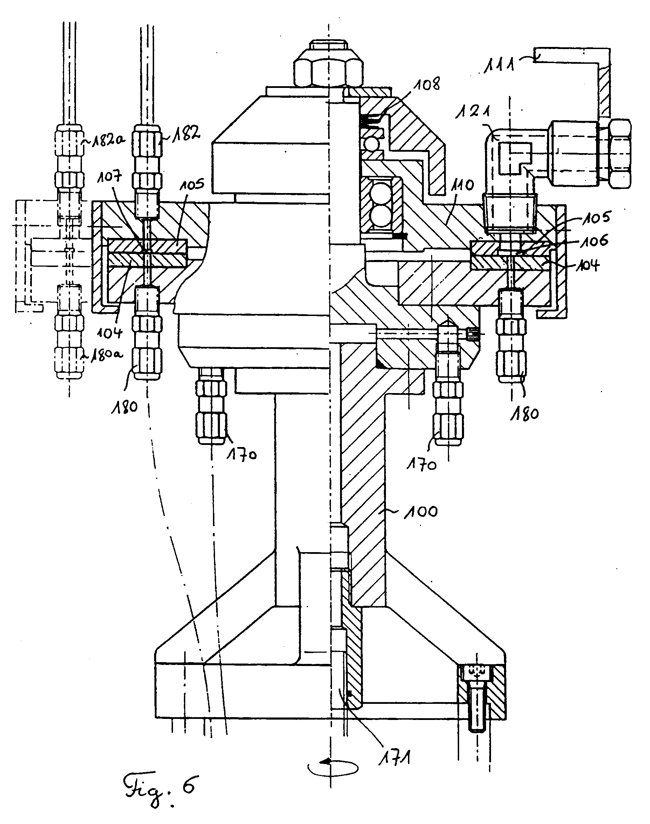

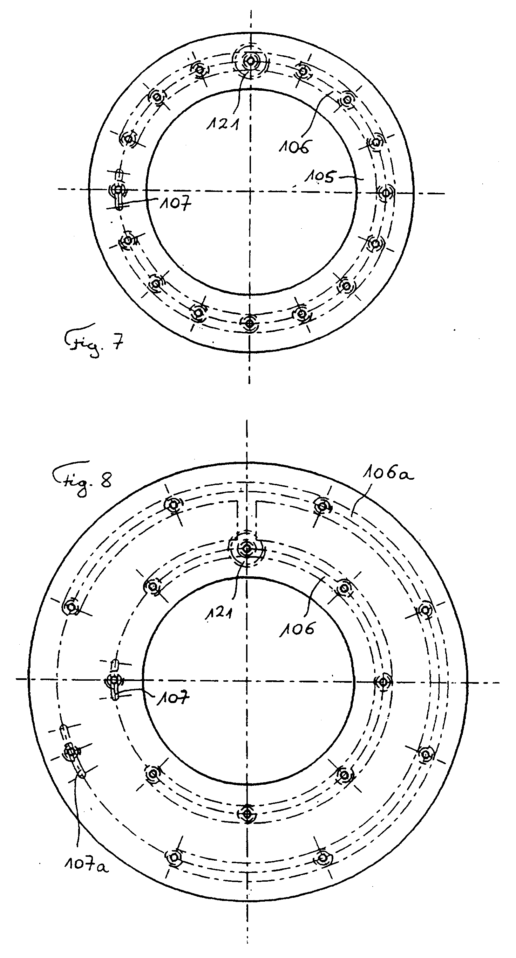

Figur 6 zeigt eine mechanische Verteileinrichtung, welche anstelle der elektrisch geschalteten Verteileinrichtung von Figur 4 auf das dort gezeigte Karussell 47 aufgesetzt ist, wobei in Figur 6 dieses Karussell nicht dargestellt ist. Die mechanische Verteileinrichtung weist einen unteren, drehenden Teil 100 auf, der mit der Drehachse des Karussells 47 verbunden ist. Dieser Teil 100 dreht synchron mit dem Karussell, bzw. mit den Flaschen. An dem drehenden Teil 100 sind eine Mehrzahl von Anschlussstücken 180 für die nicht dargestellten Leitungen 11 (Figur 4) vorgesehen. Ueber diese Anschlussstücke gelangen die Gasproben in das Innere der Verteileinrichtung. Axial oberhalb des drehenden Teils 100 ist ein feststehender Teil 110 mit einer Verdrehsicherung 111 angeordnet. Von diesem feststehenden Teil 110 führt ein Anschlusstück 121 zur nicht dargestellten Saugpumpe 26 (Figur 1), welche kontinuierlich Luft (Gasproben) aus den Flaschen absaugt. Vom feststehenden Teil führt ferner ein Anschlusstück 182 zum feststehenden Massenspektrometer 1 (in Figur 6 nicht dargestellt). Falls ein zweites Massenspektrometer 1 a vorgesehen ist, ist für dieses ein Anschlussstück 182 a vorgesehen. Der drehende Teil 100 und der feststehende Teil 101 gleiten mittels Dichtflächen 104 bzw. 105 aufeinander. In einer oder beiden der Dichtflächen 104, 105 sind die Verteilkammern vorgesehen. Eine erste Verteilkammer 106 der Dichtung 105 dient als Absaugkammer und steht ständig über das Anschlussstück 121 mit der Saugpumpe in Verbindung. Eine zweite Verteilkammer 107 dient als Durchgangskammer für die jeweils an die Prüfeinrichtung 1 abzugebende Gasprobe und steht einerseits ständig mit dem Anschlussstück 182 in Verbindung. Die Absaugkammer 106 ist kreisbogenförmig (Figur 7) und steht über Durchgangsbohrungen in Verbindung mit allen Anschlussstücken 180 bzw. Leitungen 11 bzw. Flaschen, mit Ausnahme eines einzigen Anschlussstückes 180, welches in Figur 6 in der linken Hälfte der Zeichnung gezeigt ist, das mit der Durchgangskammer 107 in Verbindung steht. Ueber dieses Anschlussstück 180 wird aus der zugehörigen Flasche über die entsprechende Leitung 11 eine Gasprobe durch die Kammer 107 und das Anschlussstück 182 an das Massenspektrometer abgegeben. Die Gasproben aus allen anderen Flaschen gelangen über die Kammer 106 und den Anschluss 121 in die Saupumpe bzw. in die Umgebungsluft. Durch die Drehbewegung des Karussells, welcher untere Teil der Verteileinrichtung folgt, gelangt nacheinander jedes Anschlussstück 180 in Verbindung mit der Kammer 107 bzw. mit dem Massenspektrometer, während jeweils die anderen Anschlussstücke 180 in Verbidnung mit der Kammer 106 stehen.

Sollen zwei Massenspektrometer 1 und 1 a im Einsatz stehen, so kann eine zweite Kammeranordnung 106 a, 107 a vorgesehen werden, wie dies in Figur strichliert angedeutet und in Figur 8 gezeigt ist. Die Ansaugkammern 106 und 106 a können dabei in Verbindung stehen, bzw. sind an denselben Saugpumpenanschluss 121 angeschlossen. Die Kammern 107, 107 a hingegen sind getrennt und je mit dem zugehörigen Massenspektrometer 1 bzw. 1 a verbunden. Auf die geschilderte Weise ergibt sich eine doppelte Verarbeitungskapazität.

Die Dichflächen 104, 105 sind als verschleissoptimierte Trockengleitpartner ausgeführt z.B. jeweils als Hartmetall-Gleitflächen oder jeweils als Kupfer-Gleitflächen oder als einerseits Hartmetall-, andererseits Keramik-Gleitfläche. Die Gleitflächen können bei der gezeigten Ausführung einfach ausgewechselt werden. Um eine gute Dichtheit zwischen den Dichtflächen 104, 105 zu erreichen, werden die beiden Teile 100, 110 der Verteileinrichtung mittels einer sphärisch gelagerten Feder 108 axial vorgespannt. Eine zentrale Befestigung erlaubt eine schnelle Montage bzw. Demontage der Verteileinrichtung.

Für die jeweils dem Massenspektrometer zugeführte Gasprobe ergibt die gezeigte Anordnung einen kurzen, geradlinigen Weg, was den Memory-Effekt vermindert.

Für die Zufuhr von Einblasluft in die Flaschen ist im gezeigten Beispiel ein zentraler Kanal 171 vorgesehen, welcher in Anschlussstücke 170 mündet, an welchen jeweils die entsprechende Leitung der flexiblen, beheizten Schläuche 10 angeschlossen sind.

## Patentansprüche

1. Verfahren zum Prüfen von entlang einer Förderstrecke geförderten Flaschen, insbesondere von Kunststoff-Flaschen, auf das Vorhandensein von Verunreinigungen, durch Prüfung von aus einzelnen Flaschen entnommenen Gasproben, wobei einer Mehrzahl von Flaschen gleichzeitig Gas entnommen wird, und dass die entnommenen Gasproben in gesteuerter Reihenfolge nacheinander an den Eingang einer mehreren Flaschen gemeinsamen Prüfeinrichtung gegeben werden,
dadurch gekennzeichnet, dass die Gasprobe als kontinuierlich während eines vorbestimmten Förderstreckenabschnittes aus der Flasche abgesaugter Gasstrom entnommen wird, der für die Prüfung an eine Verteileinrichtung geführt und von dort für vorbestimmte Zeit an die Prüfeinrichtung abgegeben wird und während der restlichen Entnahmezeit in die Umgebungsluft abgegeben wird.

2. Verfahren nach Anspruch 1, dadurch gekennzeichnet, dass als Prüfeinrichtung ein Massenspektrometer vorgesehen ist.

3. Verfahren nach einem der Ansprüche 1 oder 2 dadurch gekennzeichnet, dass während der Gasprobenentnahme Luft in die Flasche eingeblasen wird.

4. Verfahren nach einem der Ansprüche 1 bis 3, dadurch gekennzeichnet, dass vor der Prüfung der Gasprobe eine Prüfung auf das Vorhandensein von Restflüssigkeit in der Flasche erfolgt, und dass Flaschen, deren Restflüssigkeitsinhalt einen vorgewählten Grenzwert übersteigt, ausgeschieden und somit der Gasprobenprüfung entzogen werden.

5. Verfahren nach einem der Ansprüche 1 bis 4, dadurch gekennzeichnet, dass Flaschen, die aufgrund einer Prüfung auszuscheiden sind, stehend aus der Förderstrecke ausgeschieden und an eine Entsorgungsstelle gefördert werden.

6. Vorrichtung zur Durchführung des Verfahrens nach einem der Ansprüche 1 bis 5, mit einer Flaschenfördereinrichtung und mehreren, in die zu prüfenden Flaschen einführbaren Probeentnahmeorgane und mit den Probeentnahmeorganen verbundenen Leitungen, gekennzeichnet durch ein Saugmittel zum Ansaugen von Gas aus den Flaschen mittels der Probeentnahmeorgane, mindestens und eine Verteileinrichtung, welche Anschlüsse für die Leitungen, für das Saugmittel und für das Massenspektrometer aufweist.

7. Vorrichtung nach Anspruch 6, dadurch gekennzeichnet, dass die Probeentnahmeorgane von jeweils an einem vertikal verschieblichen Schlitten angeordneten Saugrohren gebildet werden.

8. Vorrichtung nach Anspruch 6 oder 7, dadurch gekennzeichnet, dass die Leitungen mindestens teilweise als beheizbare Schlauchleitungen ausgeführt sind.

9. Vorrichtung nach einem der Ansprüche 6 bis 8, dadurch gekennzeichnet, dass die Fördereinrichtung ein Karussell für die Flaschen umfasst, das mit der Verteileinrichtung gekoppelt ist, wobei durch die Drehbewegung des Karussells die Verteileinrichtung gesteuert wird.

10. Vorrichtung nach Anspruch 9, dadurch gekennzeichnet, dass die Verteileinrichtung in der Drehachse des Karussells vorgesehen ist und einen feststehenden Teil umfasst, welcher mit der Prüfeinrichtung und dem Saugmittel verbunden ist, und einen drehbaren Teil umfasst, an welchem die Leitungen zu den Probeentnahmeorganen angeschlossen sind.

11. Vorrichtung nach Anspruch 10, dadurch gekennzeichnet, dass der feststehende und der drehende Teil axial gegeneinander vorgespannt sind und mittels kreisringförmiger Dichtflächen trocken aufeinander abgestützt sind.

12. Vorrichtung nach Anspruch 11, dadurch gekennzeichnet, dass die Dichtflächen von den Trockengleitpartnern Hartmetall-Hartmetall, Kupfer-Kupfer oder Hartmetall-Keramik gebildet werden.

13. Vorrichtung nach einem der Ansprüche 6 bis 12, gekennzeichnet durch eine Einrichtung zum Einblasen von Luft in die zu prüfenden Flaschen.

14. Vorrichtung nach einem der Ansprüche 6 bis 13, dadurch gekennzeichnet, dass in Förderrichtung vor den Probeentnahmeorganen eine Flaschenhöhenprüfanordnung, eine Restmengenprüfeinrichtung eine Deckelprüfanordnung und Flaschenausscheidemittel angeordnet sind.

## Claims

1. Method for inspecting bottles being conveyed along a conveying line, more particularly bottles made of plastic, for the presence of contaminants, by testing samples of gas removed from individual bottles, in which gas is removed simultaneously from a plurality of bottles and the gas samples removed are taken one after the other in a controlled sequence to the inlet of a testing device common to a plurality of bottles, characterised in that the gas sample is removed as a stream of gas extracted continuously from the bottle over a predetermined section of the conveying line and passed to a distributing device for testing and thence is discharged to the testing device for a predetermined time and for the remainder of the sampling time is discharged into the surrounding air

2. Method according to claim 1, characterised by the provision of a mass spectrometer as the testing device.

3. Method according to claim 1 or 2, characterised in that air is blown into the bottle while the gas sample is being removed.

4. Method according to any one of claims 1 to 3, characterised in that before the gas sample is tested a check is carried out for the presence of residual liquid in the bottle, and in that bottles whose residual liquid content exceeds a preselected limit value are segregated and thus are not subjected to the gas sample test.

5. Method according to any one of claims 1 to 4, characterised in that bottles that are to be segregated as a result of an inspection are segregated upright from the conveying line and conveyed upright to a disposal point.

6. Apparatus for carrying out the method according to any one of claims 1 to 5, with a bottle conveying system and a plurality of sampling devices insertable into the bottles being inspected and with lines connected to the sampling devices, characterised by suction means for extracting gas from the bottles by means of the sampling devices, at least one mass spectrometer, and a distributing mechanism incorporating connections for the lines, for the suction means and for the mass spectrometer.

7. Apparatus according to claim 6, characterised in that the sampling devices are in each case constituted by suction tubes arranged on a vertically displaceable slide.

8. Apparatus according to claim 6 or 7, characterised in that at least some of the lines are in the form of heatable hoses.

9. Apparatus according to any one of claims 6 to 8, characterised in that the conveying system includes a carousel for the bottles which is coupled to the distributing mechanism, the distributing mechanism being controlled by the rotational movement of the carousel.

10. Apparatus according to claim 9, characterised in that the distributing mechanism is provided in the rotational axis of the carousel and includes a fixed part which is connected to the testing device and to the suction means, and a rotary part to which the lines to the sampling devices are connected.

11. Apparatus according to claim 10, characterised in that the fixed part and the rotating part are axially pressed together and are supported on one another with a dry bearing by annular sealing faces.

12. Apparatus according to claim 11, characterised in that the sealing faces are constituted by the dry sliding material combinations carbide/carbide, copper/copper or carbide/ceramics.

13. Apparatus according to any one of claims 6 to 12, characterised by a device for blowing air into the bottles being inspected.

14. Apparatus according to any one of claims 6 to 13, characterised in that a bottle height inspection arrangement, a residues testing device, a cap test arrangement and bottle segregating means are arranged before the sampling devices in the conveying direction.

## Revendications

1. Procédé pour le contrôle de bouteilles acheminées le long d'une voie de desserte, notamment de bouteilles en matière plastique, relatif à la présence d'impuretés, par examen d'échantillons de gaz prélevés dans les différentes bouteilles, dans lequel on extrait du gaz simultanément d'une multiplicité de bouteilles, et dans lequel les échantillons de gaz extraits sont amenés et pilotés successivement les uns après les autres à l'entrée d'un dispositif de contrôle commun à plusieurs bouteilles, caractérisé en ce que l'échantillon de gaz est prélevé continuellement par un courant de gaz aspiré de la bouteille sur une section prédéterminée de la voie de desserte, qui est guidé pour l'examen jusqu'à un dispositif répartiteur et de là est délivré au dispositif de contrôle pour une durée prédéterminée, et pendant la durée de prélèvement restante est rejeté dans l'air environnant.

2. Procédé selon la revendication 1, caractérisé en ce que le dispositif de contrôle est un spectromètre de masse.

3. Procédé selon la revendication 1 ou 2, caractérisé en ce que de l'air est soufflé dans la bouteille pendant l'extraction d'un échantillon de gaz.

4. Procédé selon l'une quelconque des revendications 1 à 3, caractérisé en ce qu'on effectue un contrôle de la présence de liquide résiduel dans la bouteille avant le contrôle de l'échantillon gazeux, et en ce que les bouteilles dont le contenu en liquide résiduel dépasse une valeur limite prédéterminée sont retirées et exclues du contrôle d'échantillon gazeux.

5. Procédé selon l'une des revendications 1 à 4, caractérisé en ce que les bouteilles qui ont été exclues en raison d'un contrôle, sont mises à l'écart de la voie de desserte et acheminées à un endroit de décharge.

6. Dispositif pour la mise en oeuvre du procédé selon l'une des revendications 1 à 5, comportant une installation de desserte de bouteilles, plusieurs organes de prélèvement d'échantillons pouvant être introduits dans les bouteilles à contrôler, et des conduites étant reliées aux organes de prélèvement d'échantillons, caractérisé par des moyens d'aspiration pour l'aspiration de gaz hors des bouteilles au moyen des organes de prélèvement d'échantillons, par au moins un spectromètre de masse et par un système répartiteur pourvu de raccords pour les conduites, pour des moyens d'aspiration et pour le spectromètre de masse.

7. Dispositif selon la revendication 6, caractérisé en ce que les organes de prélèvement d'échantillons sont chacun formés par des tubes d'aspiration disposés sur un chariot coulissant verticalement.

8. Dispositif selon la revendication 6 ou 7, caractérisé en ce que les conduites sont constituées au moins en partie sous forme de tuyaux souples pouvant être chauffés.

9. Dispositif selon l'une des revendications 6 à 8, caractérisé en ce que le système d'amenée comprend un carrousel pour les bouteilles accouplé au dispositif répartiteur, le mouvement de rotation du carrousel étant commandé par le système répartiteur.

10. Dispositif selon la revendication 9, caractérisé en ce que le système répartiteur est prévu dans l'axe de rotation du carrousel et comprend une partie fixe qui est reliée au dispositif de contrôle et aux moyens d'aspiration, et une partie rotative à laquelle sont reliées les conduites des organes de prélèvement d'échantillons.

11. Dispositif selon la revendication 10, caractérisé en ce que la partie fixe et la partie rotative sont mises sous tension réciproquement et s'appuient l'une sur l'autre à sec au moyen de surfaces d'étanchéité annulaires.

12. Dispositif selon la revendication 11, caractérisé en ce que les surfaces d'étanchéité sont constituées par des parties coopérantes et glissantes à sec métal dur-métal dur, cuivre-cuivre ou métal dur-céramique.

13. Dispositif selon l'une des revendications 6 à 12, caractérisé en ce qu'il comprend un système de soufflage d'air dans les bouteilles à contrôler.

14. Dispositif selon l'une des revendications 6 à 13, caractérisé en ce que sont prévus, dans la direction d'avancement, un dispositif de contrôle des hauteurs de bouteilles, un dispositif de contrôle des quantités restantes, un dispositif de contrôle des bouchons et des moyens de séparation des bouteilles.
